(19) Europäisches Patentamt
European Patent Office
Office européen des brevets

(11) EP 0 733 046 B1

(12) EUROPÄISCHE PATENTSCHRIFT

(45) Veröffentlichungstag und Bekanntmachung des Hinweises auf die Patenterteilung:
**27.06.2001 Patentblatt 2001/26**

(21) Anmeldenummer: **94931019.7**

(22) Anmeldetag: **31.10.1994**

(51) Int Cl.[7]: **C07D 239/95**, C07D 487/04, C07D 213/74, C07D 295/14, C07D 239/42, C07D 241/20, C07D 403/04, A61K 31/505

(86) Internationale Anmeldenummer:
**PCT/EP94/03581**

(87) Internationale Veröffentlichungsnummer:
**WO 95/12584 (11.05.1995 Gazette 1995/20)**

(54) **BENZOYLGUANIDINE, IHRE HERSTELLUNG UND IHRE VERWENDUNG IN ARZNEIMITTELN**

BENZOYL GUANIDINES, THEIR PRODUCTION AND THEIR USE IN MEDICAMENTS

BENZOYLGUANIDINES, LEUR PREPARATION ET LEUR UTILISATION DANS DES MEDICAMENTS

(84) Benannte Vertragsstaaten:
**AT BE CH DE DK ES FR GB GR IE IT LI LU MC NL PT SE**

(30) Priorität: **04.11.1993 DE 4337611**

(43) Veröffentlichungstag der Anmeldung:
**25.09.1996 Patentblatt 1996/39**

(73) Patentinhaber:
- **Boehringer Ingelheim Pharma KG**
**55218 Ingelheim am Rhein (DE)**
Benannte Vertragsstaaten:
**BE CH DE DK ES FR GR IT LI LU MC NL PT SE AT**
- **Boehringer Ingelheim International GmbH**
**55218 Ingelheim am Rhein (DE)**
Benannte Vertragsstaaten:
**GB IE**

(72) Erfinder:
- **ROOS, Otto**
**D-55270 Schwabenheim (DE)**
- **SPECK, Georg**
**D-55218 Ingelheim (DE)**
- **LÖSEL, Walter**
**D-55435 Gau-Algesheim (DE)**
- **ARNDTS, Dietrich**
**D-55437 Appenheim (DE)**
- **BECHTEL, Wolf-Dietrich**
**D-55437 Appenheim (DE)**

(74) Vertreter: **Laudien, Dieter, Dr.**
**Boehringer Ingelheim GmbH,**
**Binger Strasse 173**
**55216 Ingelheim (DE)**

(56) Entgegenhaltungen:
**EP-A- 0 416 499** **EP-A- 0 556 672**

Bemerkungen:
Die Akte enthält technische Angaben, die nach dem Eingang der Anmeldung eingereicht wurden und die nicht in dieser Patentschrift enthalten sind.

EP 0 733 046 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft neue Benzoylguanidine, ihre Herstellung nach konventionellen Methoden und ihre Verwendung in bzw. bei der Herstellung von Arzneimitteln.

Die neuen Verbindungen entsprechen der Formel

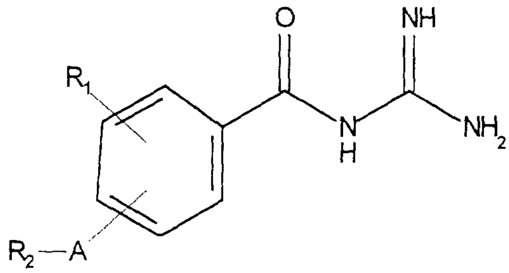

(I)

und können als Basen oder als Salze mit Säuren vorliegen.

In der Formel I steht

A für

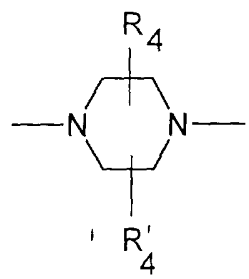

$R_1$ für R' - $SO_2$ - oder R' - NH - $SO_2$ - (R' gleich $C_1$-$C_5$-Alkyl, halogen- oder phenylsubstituiertes $C_1$-$C_5$-Alkyl, wobei der Phenylrest bis zu drei Substituenten aus der Gruppe Halogen, $C_1$-$C_4$-Alkyl oder $C_1$-$C_4$-Alkoxy aufweisen kann), F, Cl oder $CF_3$;

$R_2$ für einen Rest der Formel

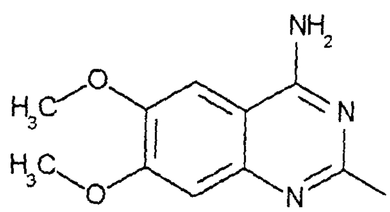

(II)

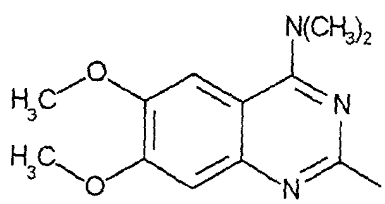

(III)

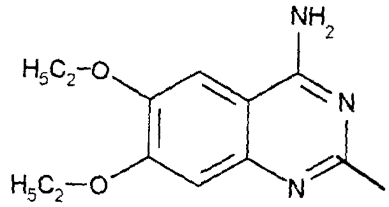

(IV)

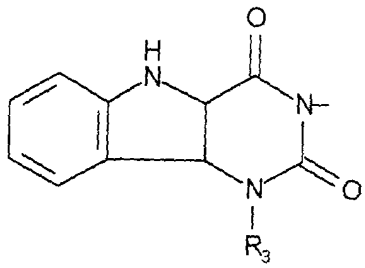

(V)

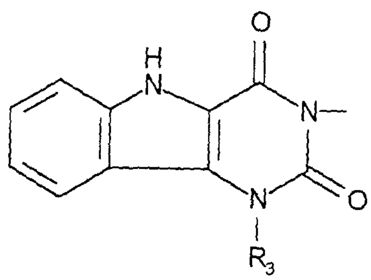

(VI)

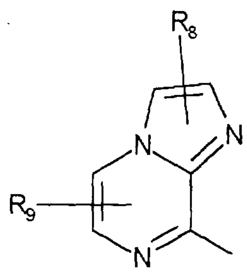

(VII)

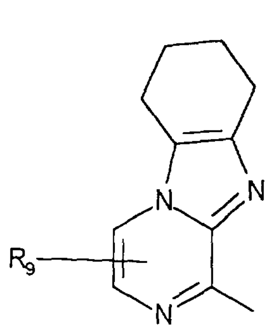

(VIII)

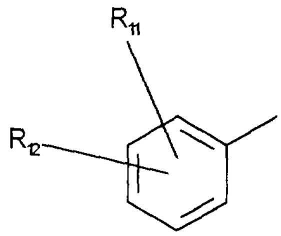

(IX)

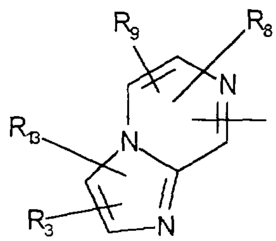

(X)

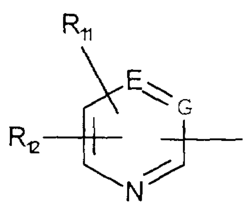

(XI)

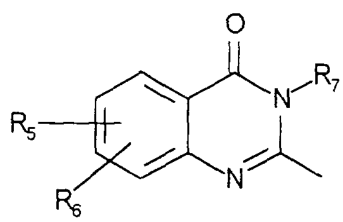

(XII)

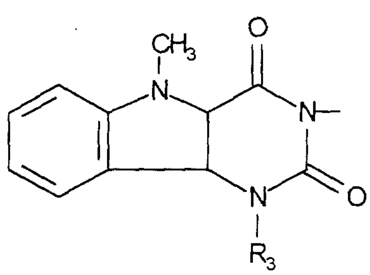

(XIII)

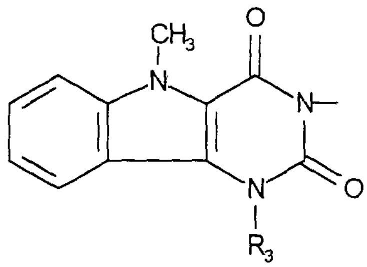

(XIV)

worin

| | |
|---|---|
| $R_3$, $R_4$ und $R_4$', | , die gleich oder verschieden sein können, Wasserstoff, $C_1$-$C_4$-Alkyl, $R_4$ und $R_4$' auch Phenyl, Benzyl und $C_3$-$C_7$-Cycloalkyl, |
| $R_5$ und $R_6$, | die gleich oder verschieden sein können, Wasserstoff, Methyl, Methoxy, Hydroxy oder Halogen, |
| $R_7$ | Wasserstoff, $C_1$-$C_4$-Alkyl, Benzyl oder Benzyloxy, |
| $R_8$ und $R_9$, | die gleich oder verschieden sein können, Wasserstoff, $C_1$-$C_4$-Alkyl, Phenyl oder Halogen, |
| $R_{10}$ | Wasserstoff oder $C_1$-$C_6$-Alkyl, das auch durch Phenyl oder methyl-, methoxy- oder halogensubstituiertes Phenyl substituiert sein kann, |
| $R_{11}$ und $R_{12}$, | die gleich oder verschieden sein können, Wasserstoff, Methyl, Methoxy, Phenyl, Benzyl, Nitro, Cyano, Halogen, Trifluormethyl, Amino, $NR_{10}R_{13}$, 1-Pyrrolidinyl, 1-Pyrazolinyl, 1-Imidazolidinyl, 1-Piperidinyl, 1Piperazinyl oder Carbamoyl, $R_{11}$ auch einen ankondensierten Benzolring, der bis zu drei Substituenten aus der Gruppe Methyl, Methoxy, Halogen, $CF_3$ und CN aufweisen kann, |
| $R_{13}$ | Wasserstoff, $C_1$-$C_4$-Alkyl, das auch durch Phenyl, Phenoxy oder Benzyloxy substituiert sein kann, oder Halogen,und |
| E und G, | die gleich oder verschieden sein können, N oder CH bedeuten. |

Die in den obigen Definitionen genannten Alkylgruppen können geradkettig oder verzweigt mit 1 bis 4, insbesondere 1 bis 3, vor allem 1 bis 2 C-Atomen sein. Von den Halogenen sind Fluor, Chlor und Brom, vor allem Fluor und Chlor bevorzugt.

Soweit die neuen Verbindungen in verschiedenen stereoisomeren bzw. cis-/trans-isomeren Formen existieren können, sind mit den obigen Formeln die reinen Formen wie auch ihre Mischungen gemeint.

Die Gruppen $R_2$-A [= $R_2$-(1-Piperazin-4-yl)] haben typischerweise Strukturen wie die folgenden:

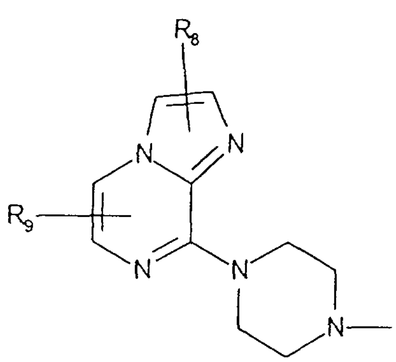

(VII')

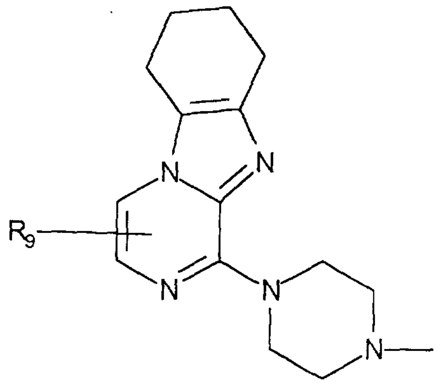

(VIII')

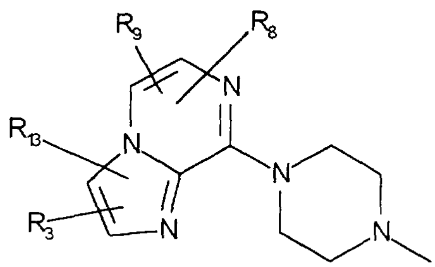

(X')

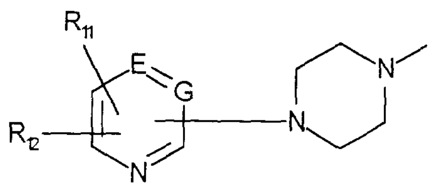

(XI')

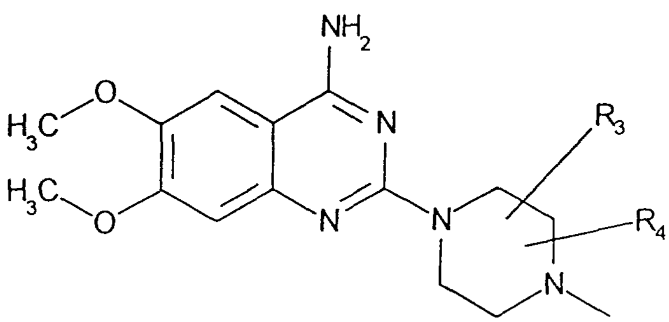

(XII')

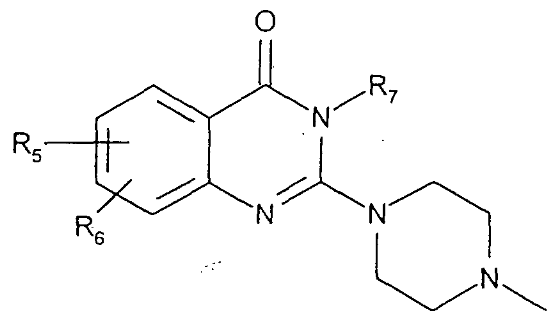

(XIV')

Die neuen Verbindungen werden nach üblichen Methoden erhalten, insbesondere nach den folgenden Verfahren.

1. Umsetzung einer Verbindung der Formel

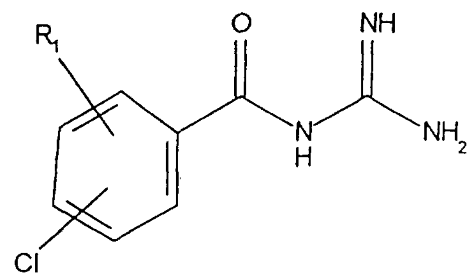

(XVI)

mit einem Amin der Formel

$$R_2 - A - H \quad \text{(XVII)}$$

wobei $R_1$ und $R_2$ die oben angegebene Bedeutung haben.

Die Umsetzung erfolgt in einem polaren, möglichst wasserfreien Lösungsmittel oder Lösungsmittelgemisch, insbesondere Dimethylsulfoxid, Dimethylformamid, vorzugsweise in Gegenwart einer Base, etwa Triethylamin, N-Methylpiperidin, Pyridin, in der Wärme.

2. Zur Herstellung solcher Verbindungen, in denen $R_2$ einen Rest der Formel II, III oder IV darstellt, kann die Verknüpfung auch über ein anderes Stickstoffatom des Restes $R_2$ erfolgen. Beispielsweise werden die Verbindungen mit $R_2$ gleich II auch erhalten, indem man ein Amin der Formel

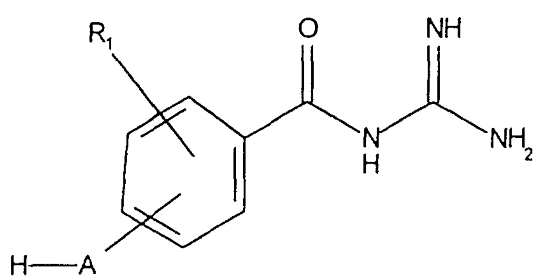

(XVIII)

(worin $R_1$ die obige Bedeutung hat), mit dem Chinazolinonderivat der Formel

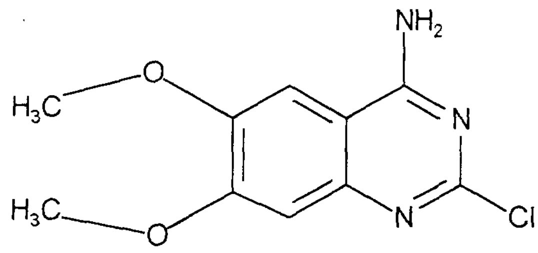

(XIX)

wie unter 1. beschrieben, umsetzt.

3. Umsetzung einer Verbindung der Formel

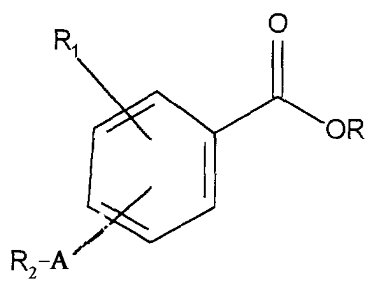

(XX)

(R Alkylrest, Benzyl)

mit Guanidin. Die Umsetzung ist nicht auf die Ester mit R in den genannten Bedeutungen beschränkt, doch wird der Fachmann zweckmäßig einen gut herstellbaren Ester, etwa den Methyl- oder Ethylester verwenden, bzw. einen Ester, bei dessen Umsetzung ein unproblematischer Alkohol entsteht.

Bevorzugt verwendet man den Alkohol, der auch in der Estergruppe enthalten ist, indem man z.B. einen Methylester der Formel XX in Methanol bei Siedetemperatur umsetzt. Dieses Verfahren eignet sich am besten zur Herstellung der erfindungsgemäßen Verbindungen.

Soweit die Verbindungen in stereoisomeren Formen vorliegen können, werden entsprechende Ausgangsprodukte eingesetzt oder es werden ggf. bei der Herstellung gebildete Mischungen in die Komponenten aufgetrennt.

Die Ausgangsstoffe können, soweit sie nicht schon bekannt sind, ebenfalls nach konventionellen Verfahren erhalten werden. Werden beispielsweise anstelle der N-Amidino-carboxamide der Formel XVI bzw. der Formel XVII die entsprechenden Ester eingesetzt, so gelangt man zu den Ausgangsstoffen der Formel XX.

Die Verbindungen der Formel I sind als Wirkstoffe in Arzneimitteln verwendbar oder können als Zwischenprodukte zur Herstellung solcher Wirkstoffe Verwendung finden. Unter anderem hemmen die neuen Verbindungen den $Na^+/H^+$- und den $Na^+/Li^+$-Austausch. Die erfindungsgemäßen Wirkstoffe können als Antihypertensiva, Mucolytika, Diuretika und Cancerostatika benutzt werden; sie sind ferner anwendbar bei Krankheiten, die im Zusammenhang mit Ischämien stehen (Beispiele: cardiale, cerebrale, gastrointestinale, pulmonale, renale Ischämie, Ischämie der Leber, Ischämie der Skelettmulskulatur). Entsprechende Krankheiten sind beispielsweise coronare Herzkrankheit, Angina pectoris, Embolie im Lungenkreislauf, akutes oder chronisches Nierenversagen, chronische Niereninsuffizienz, Hirninfarkt (z. B. nach der Wiederdurchblutung von Hirnarealen nach Auflösung von Gefäßverschlüssen, auch in Kombination mit t-PA, Streptokinases, Urokinase usw.), akute und chronische Durchblutungsstörungen des Hirns. Bei der Reperfusion des ischämischen Herzens (z.B. nach einem Anginapectoris-Anfall oder einem Herzinfarkt) können irrevesible Schädigungen an Cardiomyocyten in der betroffenen Region auftreten. Die erfindungsgemäßen Verbindungen können u.a. in einem solchen Fall zur Cardioprotektion benutzt werden. Hervorzuheben sind auch die geringen Nebenwirkungen der neuen Verbindungen, insbesondere auch auf die $a_{1-}$ und/oder $a_2$-Rezeptoren.

In das Anwendungsgebiet Ischämie ist auch die Verhinderung von Schäden an Transplantaten einzubeziehen (z. B. als Schutz des Transplantats vor, während und nach der Implantation), die im Zusammenhang mit Transplantationen auftreten können.

Zur Anwendung der Wirkstoffe eignen sich übliche Formulierungen, etwa Tabletten, Dragées, Kapseln, Granulate, Injektionslösungen, ggf. auch nasal applizierbare Zubereitungen, wobei die Menge der Wirksubstanz in einer Einzelgabe im allgemeinen 1 bis 200 mg, vorzugsweise 20-100 mg beträgt.

Die Herstellung dieser Arzneimittelformen erfolgt in an sich bekannter Weise.

Beispiele

1. Tabletten (Zusammensetzung)

| | |
|---|---|
| Verbindung nach Beispiel | 40,0 mg |
| Maisstärke | 144,0 mg |
| sek. Calciumphosphat | 115,0 mg |
| Magnesiumstearat | 1,0 mg |
| | 300,0 mg |

2. Gelatinekapseln

Der Inhalt einer Kapsel besteht aus 50,0 mg einer Verbindung gemäß der Erfindung und 150,0 mg Maisstärke.
Die Verbindungen der nachfolgenden Tabellen liegt die nachstehende Formel (1 a) zugrunde:

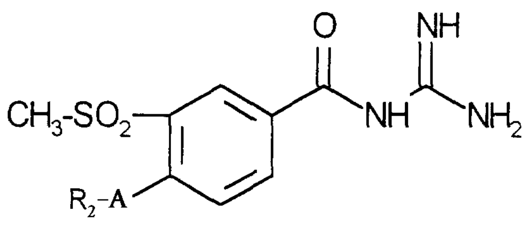

(I a)

Die nachstehenden Synthesebeispiele sollen die Erfindung näher erläutern.

Beispiel 1

N-[2-(4-Amino-6,7-dimethoxy)chinazolinyl]-N'-[1-[4-(N-amidino-carbamoyl)-2 methyl-sulfonyl]phenyl-N,N'-piperazin-Dihydrochlorid

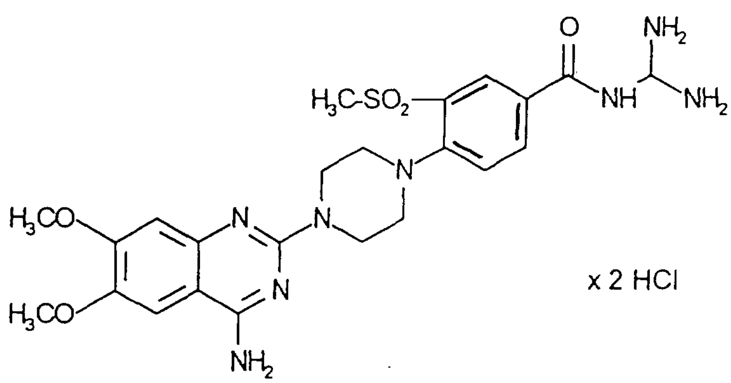

x 2 HCl

a) 5,8 g (19,44 m Mol) 4-Piperazinyl-3-methyl-sulfonylbenzoesäuremethylester, 4,65 g (19,44 m Mol) 1-Amino-3-chlor-6,7-dimethoxychenazolin und 2.02 g (20 m Mol) Triethylamin werden in 115 ml n-Amylalkohol 1,5 Std. am Rückfluß gekocht. Nach Abkühlen auf Zimmertemperatur wird von dem auskristallisierten Reaktionsprodukt abgesaugt (6,37g)

b) Zu einer Guanidinlösung, erhalten aus 1,91 g (20 m Mol) GuanidinHydrochlorid und 20 m Mol NaH in 40 ml

Dimethylformamid, werden 2,06 g (4 m Mol) des unter a) erhaltenen Esters zugesetzt und 2 Std. unter $N_2$-Atmosphäre auf 100-110°C erhitzt. Nach Abkühlen auf Zimmertemperatur wird vom Ungelösten abfiltriert und das Filtrat im Vakuum zur Trockne eingedampft. Der Rückstand wird über eine Kieselgelsäule mit einer Lösung aus Essigester: i-Propanol:Ammoniak = 70:30:5 als Eluat chromatographisch gereinigt.
In salzsaurer methanolischer Lösung werden 1,2 g der Titelverbindung vom Fp: 240-243°C erhalten.

Die in den folgenden Tabellen aufgeführten Verbindungen können entsprechend den vorstehenden Beispielen und/oder den Angaben in der Beschreibung erhalten werden.

## Tabelle 1

Verbindungen der Formel I a, worin
$R_2$-A [= $R_2$-(1-Piperazin-4-yl)] für einen Rest der Formel VII'a steht:

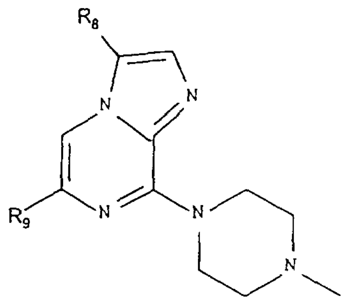

(VII'a)

| Nr. | $R_8$ | $R_9$ | Fp. [°C] |
|---|---|---|---|
| 1 | H | H | |
| 2 | $CH_3$ | Cl | |
| 3 | Cl | Cl | |
| 4 | $CH_3$ | $CH_3$ | |

## Tabelle 2

Verbindungen der Formel I a, worin
$R_2$-A [= $R_2$-(1-Piperazin-4-yl)] für einen Rest der Formel VIII'a steht:

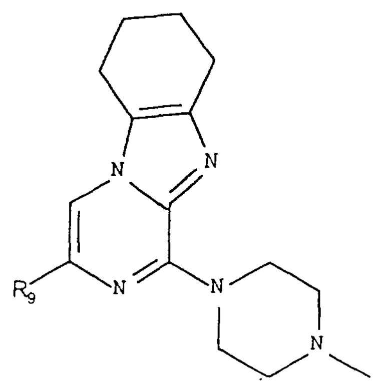

(VIII'a)

| Nr. | $R_9$ | Fp. [°C] |
|---|---|---|
| 1 | H | |
| 2 | Cl | |
| 3 | $CH_3$ | |

**Tabelle 3**

Verbindungen der Formel I a, worin

$R_2$-A [= $R_2$-(1-Piperazin-4-yl)] einen Rest der Formel X'a:

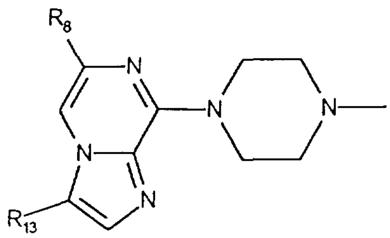

(X'a)

darstellt ($R_3$ und $R_9$ gleich H)

| Nr. | $R_8$ | $R_{13}$ | Fp. [°C] |
|---|---|---|---|
| 1 | H | H | |
| 2 | $C_2H_5$ | H | |
| 3 | H | $CH_3$ | |
| 4 | i-$C_3H_7$ | H | |
| 5 | i-$C_3H_7$ | $CH_3$ | |
| 6 | H | Br | |
| 7 | Br | H | |
| 8 | $CH_3$ | H | |

## Tabelle 3a

Verbindungen der Formel I a, worin $R_2$-A [= $R_2$-(1-Piperazin-4-yl)] einen Rest der Formel X'b:

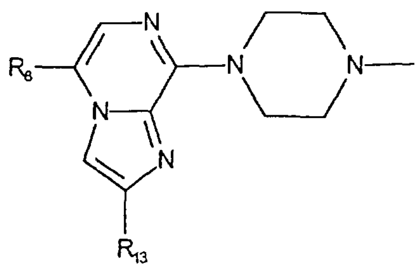

(X'b)

darstellt ($R_3$ und $R_9$ gleich H)

| Nr. | $R_8$ | $R_{13}$ | Fp. [°C] |
|---|---|---|---|
| 1 | H | H | |
| 2 | $C_2H_5$ | H | |
| 3 | H | $CH_3$ | |
| 4 | i-$C_3H_7$ | H | |
| 5 | i-$C_3H_7$ | $CH_3$ | |
| 6 | H | Br | |
| 7 | Br | H | |
| 8 | $CH_3$ | H | |

## Tabelle 4

Verbindungen der Formel I a, worin $R_2$-A [= $R_2$-(1-Piperazin-4-yl)] für einen Rest der Formel XI'a steht:

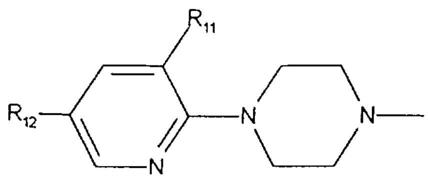

(XI'a)

| Nr. | $R_{11}$ | $R_{12}$ | Fp. [°C] |
|---|---|---|---|
| 1 | $NH_2$ | H | |
| 2 | $CONH_2$ | H | |
| 3 | Cl | H | |
| 4 | Cl | Cl | |
| 5 | H | H | |
| 6 | H | $CH_3$ | |
| 7 | $CH_3$ | H | |
| 8 | $CH_3$ | $CH_3$ | |

## Tabelle 5

Verbindungen der Formel I a, worin $R_2$-A [= $R_2$-(1-Piperazin-4-yl)] für einen Rest der Formel XII'a:

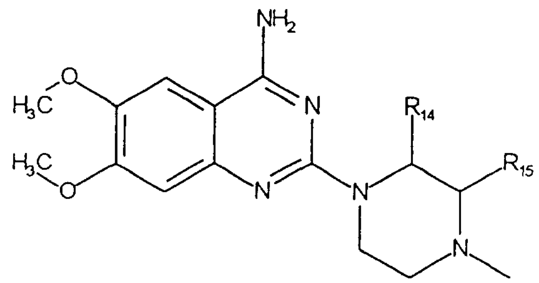

(XII'a)

steht, in der $R_{14}$ bzw. $R_{15}$ die Reste $R_3$ bzw. $R_4$ bedeuten

| Nr. | $R_{14}$ | $R_{15}$ | Form | Fp. [°C] |
|---|---|---|---|---|
| 1 | H | $CH_3$ | | |
| 2 | $CH_3$ | H | | |
| 3 | $CH_3$ | $CH_3$ | cis | |
| 4 | $CH_3$ | $CH_3$ | trans | |
| 5 | $C_2H_5$ | $CH_3$ | cis/trans | |
| 6 | $C_2H_5$ | $C_2H_5$ | cis | |
| 7 | $C_2H_5$ | $C_2H_5$ | trans | |
| 8 | H | H | | 240-3 |
| 9 | H | i-$C_3H_7$ | | |

Tabelle 6

| Verbindungen der Formel I a, worin $R_2$-A [= $R_2$-(1-Piperazin-4-yl)] für einen Rest der Formel XIV' steht, wobei sich die Positionsangaben für $R_5$ und $R_6$ auf das Chinazolinonringsystem beziehen | | | | |
|---|---|---|---|---|
| Nr. | $R_7$ | $R_5$ | $R_6$ | Fp.[°C] |
| 1 | H | H | H | |
| 2 | H | 6-$OCH_3$ | 7-$OCH_3$ | |
| 3 | H | 8-$OCH_3$ | H | |
| 4 | H | 6-OH | 7-OH | |
| 5 | H | 7-OH | H | |
| 6 | H | 8-OH | H | |
| 7 | H | 6-Cl | H | |

Tabelle 6 (fortgesetzt)

| Verbindungen der Formel I a, worin $R_2$-A [= $R_2$-(1-Piperazin-4-yl)] für einen Rest der Formel XIV' steht, wobei sich die Positionsangaben für $R_5$ und $R_6$ auf das Chinazolinonringsystem beziehen | | | | |
|---|---|---|---|---|
| Nr. | $R_7$ | $R_5$ | $R_6$ | Fp.[°C] |
| 8 | H | 6-$CH_3$ | 7-$CH_3$ | |
| 9 | $CH_3$ | H | H | |
| 10 | $CH_3$ | 6-$OCH_3$ | 7-$OCH_3$ | |
| 11 | $CH_3$ | 8-$OCH_3$ | H | |
| 12 | $CH_3$ | 6-OH | 7-OH | |
| 13 | $CH_3$ | 7-OH | H | |
| 14 | $CH_3$ | 8-OH | H | |
| 15 | $CH_3$ | 6-Cl | H | |
| 16 | $CH_3$ | 6-$CH_3$ | 7-$CH_3$ | |

Tabelle 8

| Verbindungen der Formel I a mit verschiedenen Resten $R_2$ und A gleich 1,4-Piperazindiyl | | |
|---|---|---|
| Nr. | $R_2$ | Fp.[°C] |
| 1 | Phenyl | 270-3 |
| 2 | 2-Pyrimidinyl | 200-3 |
| 3 | 2-Pyrazinyl | 245-8 |

**Patentansprüche**

**1.** Verbindungen der Formel

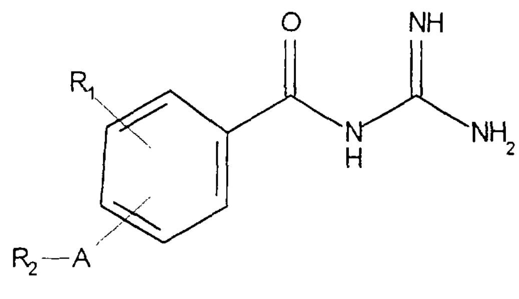

(I)

in der

A für

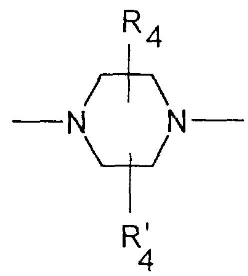

$R_1$ für $R'-SO_2-$ oder $R'-NH-SO_2-$ (R' gleich $C_1$-$C_5$-Alkyl, halogen- oder phenylsubstituiertes $C_1$-$C_5$-Alkyl, wobei der Phenylrest bis zu drei Substituenten aus der Gruppe Halogen, $C_1$-$C_4$-Alkyl oder $C_1$-$C_4$-Alkoxy aufweisen kann), F, Cl oder $CF_3$;

$R_2$ für einen Rest der Formel

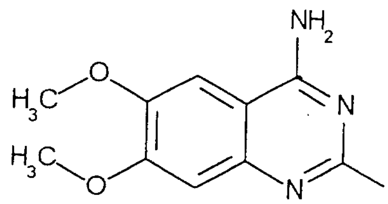

(II)

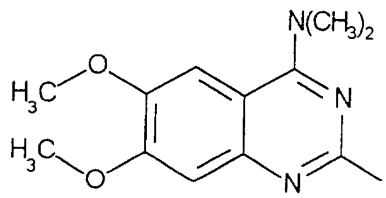

(III)

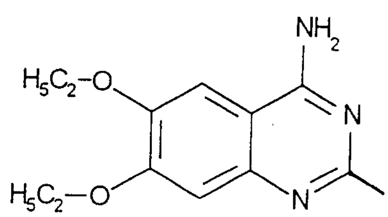

(IV)

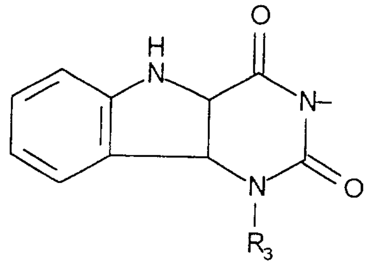

(V)

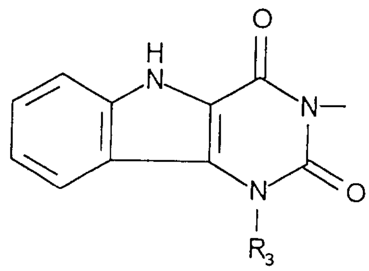

(VI)

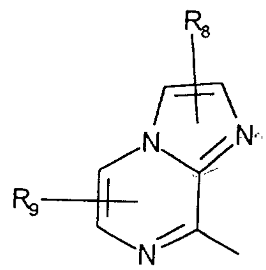

(VII)

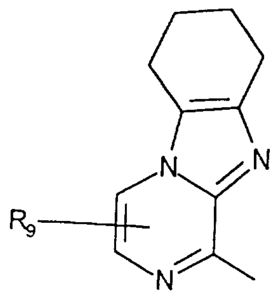

(VIII)

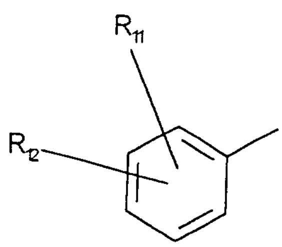

(IX)

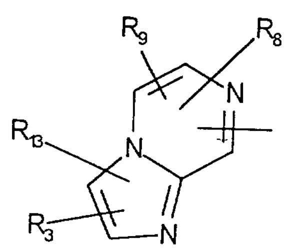

(X)

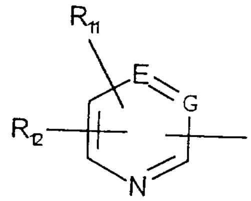

(XI)

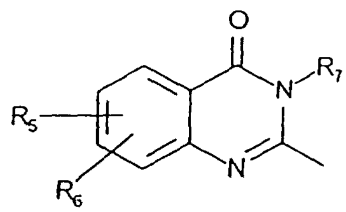

(XII)

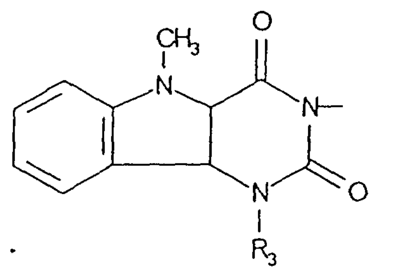

(XIII)

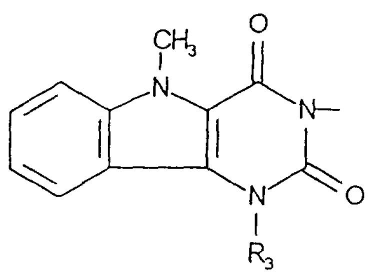

(XIV)

stehen, worin

| | |
|---|---|
| $R_3$, $R_4$ und $R_4$', | , die gleich oder verschieden sein können, Wasserstoff, $C_1$-$C_4$-Alkyl, $R_4$ und $R_4$' auch Phenyl, Benzyl und $C_3$-$C_7$-Cycloalkyl, |
| $R_5$ und $R_6$, | , die gleich oder verschieden sein können, Wasserstoff, Methyl, Methoxy, Hydroxy oder Halogen, |
| $R_7$ | Wasserstoff, $C_1$-$C_4$-Alkyl, Benzyl oder Benzyloxy, |
| $R_8$ und $R_9$, | die gleich oder verschieden sein können, Wasserstoff, $C_1$-$C_4$-Alkyl, Phenyl oder Halogen, |
| $R_{10}$ | Wasserstoff oder $C_1$-$C_6$-Alkyl, das auch durch Phenyl oder methyl-, methoxy- oder halogensubstituiertes Phenyl substituiert sein kann, |
| $R_{11}$ und $R_{12}$, | die gleich oder verschieden sein können, Wasserstoff, Methyl, Methoxy, Phenyl, Benzyl, Nitro, Cyano, Halogen, Trifluormethyl, Amino, $NR_{10}R_{13}$, 1-Pyrrolidinyl, 1-Pyrazolinyl, 1-Imidazolidinyl, 1-Piperidinyl, 1-Piperazinyl oder Carbamoyl, $R_{11}$ auch einen ankondensierten Benzolring, der bis zu drei Substituenten aus der Gruppe Methyl, Methoxy, Halogen, $CF_3$ und CN aufweisen kann, |

$R_{13}$ Wasserstoff, $C_1$-$C_4$-Alkyl, das auch durch Phenyl, Phenoxy, Benzyloxy substituiert sein kann, oder Halogen,

E und G, die gleich oder verschieden sein können, N oder CH,

bedeuten, wobei
die Verbindungen der Formel I als Basen oder als Salze und ggf. in Form der einzelnen Stereoisomeren oder cis-/trans-Isomeren bzw. der Mischungen solcher Isomeren vorliegen können.

**2.** Verbindungen der Formel I nach Anspruch 1, in der $R_2$ einen Rest der Formel II bis XV, $R_3$, $R_4$ und $R_4'$, die gleich oder verschieden sein können, Wasserstoff oder einen $C_1$-$C_3$-Alkylrest bedeuten.

**3.** Verbindungen der Formel I nach Anspruch 1, in denen $R_2$ einen Rest der Formel XI bedeutet, E oder G für ein Stickstoffatom stehen.

**4.** Verbindungen der Formel I a

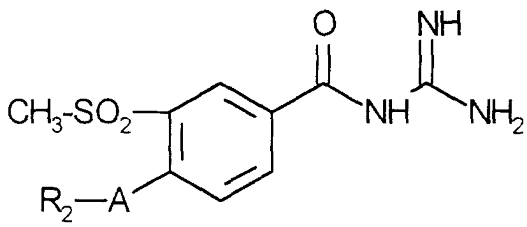

(I a)

gemäß einem der Ansprüche 1 bis 3, in der A und $R_2$ die obige Bedeutung haben.

**5.** Arzneimittel, gekennzeichnet durch einen Gehalt an einer Verbindung nach Anspruch 1 bis 4, neben üblichen Hilfs- und/oder Trägerstoffen.

**6.** Verwendung von Verbindungen nach Anspruch 1 bis 4 bei der Herstellung von Arzneimitteln.

**7.** Verbindungen nach Anspruch 1 bis 4 zur Verwendung bei Behandlung von Krankheiten, die im Zusammenhang mit Ischämien stehen, bei Hirninfarkt, akuten und chronischen Durchblutungsstörungen des Hirns, zur Cardioprotektion und zur Verhinderung von Schäden an Transplantaten.

**8.** Verfahren zur Herstellung der Verbindungen nach Anspruch 1 bis 4 nach konventionellen Methoden, dadurch gekennzeichnet, daß man

(a) eine Verbindung der Formel

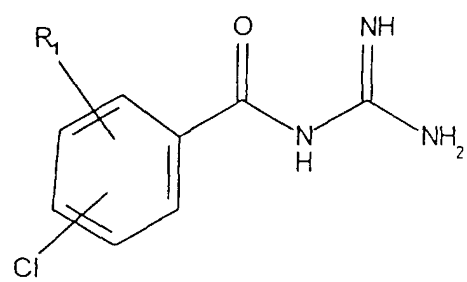

(XVI)

mit einem Amin der Formel

$R_2$ - A - H (XVII)

worin $R_1$ und $R_2$ die obige Bedeutung haben, umsetzt,
oder daß man
(b) eine Verbindung der Formel

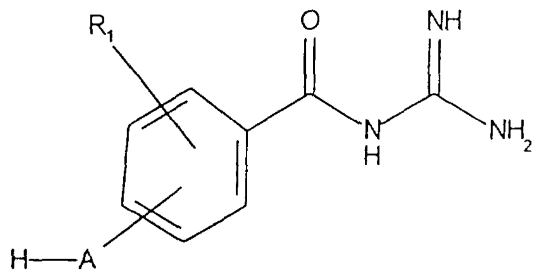

(XVIII)

worin $R_1$ die obige Bedeutung hat,
mit einer Verbindung der Formel

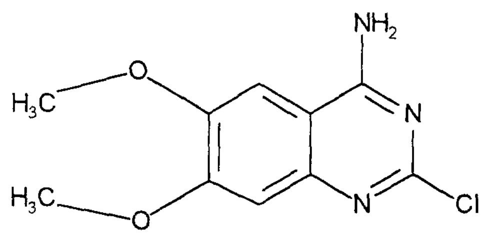

(XIX)

umsetzt,
oder daß man
(c) eine Verbindung der Formel

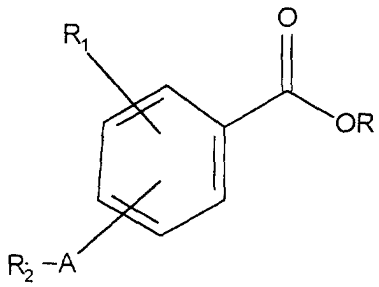

(X)

worin R einen niederen Alkylrest bedeutet,
mit Guanidin umsetzt

und die erhaltenen Produkte ggf. in sterisch unterschiedliche Formen auftrennt und/oder gewünschtenfalls erhaltene Basen mit Säuren in Salze überführt bzw. erhaltene Salze in freie Basen.

## Claims

**1.** Compounds of the formula

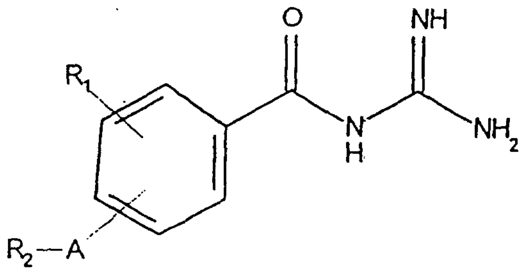

(I)

wherein

A denotes

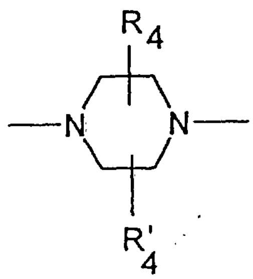

$R_1$ denotes R'-$SO_2$- or R'-NH-$SO_2$- (R' being identical to $C_{1-5}$-alkyl, halogen- or phenyl-substituted $C_{1-5}$-alkyl, whilst the phenyl group may contain up to three substituents selected from the group halogen, $C_{1-4}$-alkyl or $C_{1-4}$-alkoxy), F, CL or $CF_3$;

$R_2$ denotes a group of formula

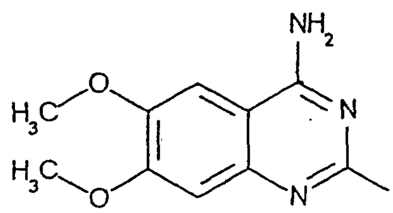

(II)

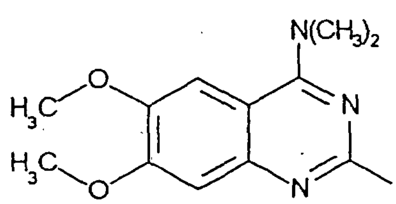

(III)

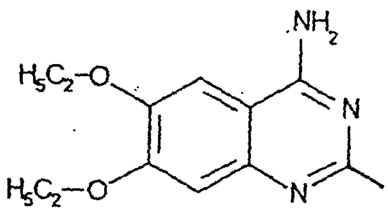

(IV)

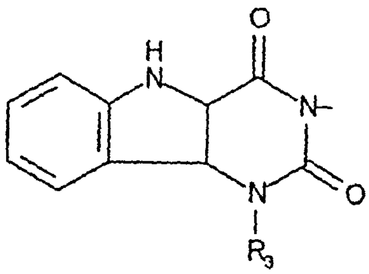

(V)

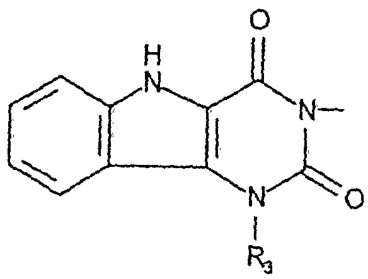

(VI)

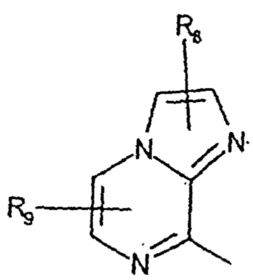

(VII)

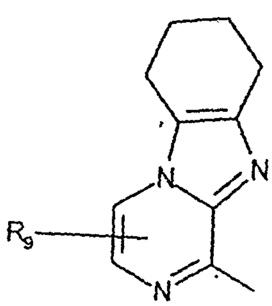

(VIII)

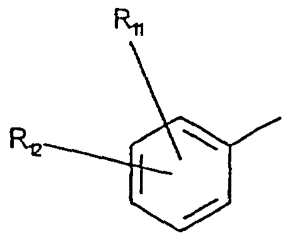

(IX)

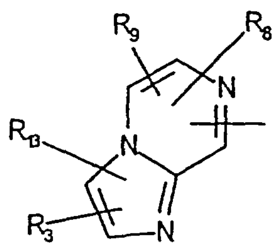

(X)

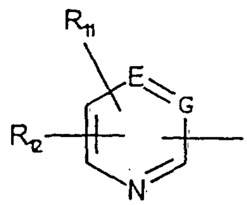

(XI)

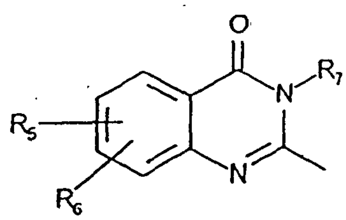

(XII)

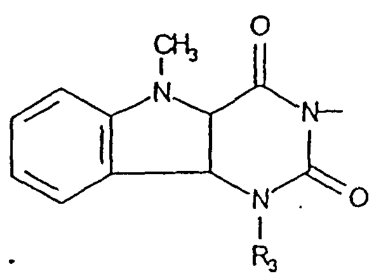

(XIII)

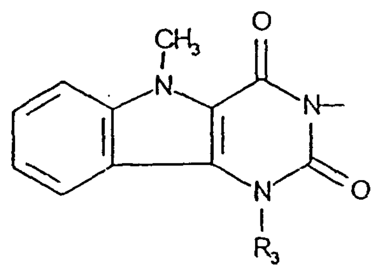

(XIV)

wherein

$R_3$, $R_4$ and $R_4$', which may be identical or different, denote hydrogen, $C_{1-4}$-alkyl, $R_4$ and $R_4$' also denote phenyl, benzyl and $C_{3-7}$-cycloalkyl,

$R_5$ and $R_6$, which may be identical or different, denote hydrogen, methyl, methoxy, hydroxy or halogen,

$R_7$ denotes hydrogen, $C_{1-4}$-alkyl, benzyl or benzyloxy,

$R_8$ and $R_9$, which may be identical or different, denote hydrogen, $C_{1-4}$-alkyl, phenyl or halogen,

$R_{10}$ denotes hydrogen or $C_{1-6}$-alkyl, which may also be substituted by phenyl or methyl-, methoxy- or halogen-substituted phenyl,

$R_{11}$ and $R_{12}$, which may be identical or different, denote hydrogen, methyl, methoxy, phenyl, benzyl, nitro, cyano, halogen, trifluoromethyl, amino, $NR_{10}R_{13}$, 1-pyrrolidinyl, 1-pyrazolinyl, 1-imidazolidinyl, 1-piperidinyl, 1-piperazinyl or carbamoyl, $R_{11}$ may also denote a fused-on benzene ring which may have up to 3 substituents selected from among methyl, methoxy, halogen, $CF_3$ and CN,

$R_{13}$ denotes hydrogen, $C_{1-4}$-alkyl, which may also be substituted by phenyl, phenoxy or benzyloxy, or halogen, and

E and G, which may be identical or different, denote N or CH, whilst

the compounds of formula I may occur as bases or as salts and optionally in the form of the individual stereoisomers or cis/trans-isomers or mixtures of such isomers.

**2.** Compounds of formula I according to claim 1, wherein $R_2$ denotes a group of formulae II to XV, $R_3$, $R_4$ and $R_4$', which may be identical or different, denote hydrogen or a $C_{1-3}$-alkyl group.

**3.** Compounds of formula I according to claim 1, wherein $R_2$ is a group of formula XI, E or G represents a nitrogen atom.

**4.** Compounds of the formula Ia

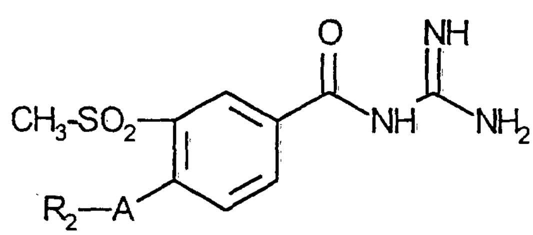

(I a)

according to one of claims 1 to 3, wherein A and $R_2$ are as hereinbefore defined.

**5.** Pharmaceutical compositions, characterised in that they contain a compound according to claims 1 to 4, together with conventional excipients and/or carriers.

**6.** Use of compounds according to claims 1 to 4 in the production of pharmaceutical compositions.

**7.** Compounds according to claims 1 to 4 for use in the treatment of diseases connected with ischaemia, in cerebral infarct, acute and chronic circulatory disorders of the brain, for cardioprotection and for preventing damage to transplants.

**8.** Process for preparing the compounds according to claims 1 to 4 by conventional methods, characterised in that

(a) a compound of formula

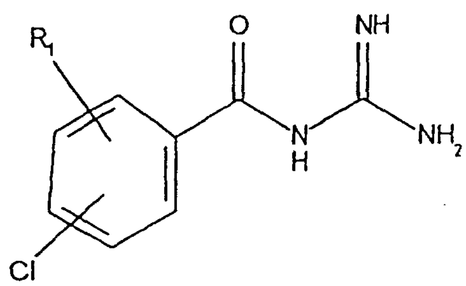

(XVI)

is reacted with an amine of formula

$$R_2 - A - H \qquad (XVII)$$

wherein $R_1$ and $R_2$ are as hereinbefore defined
or

(b) a compound of formula

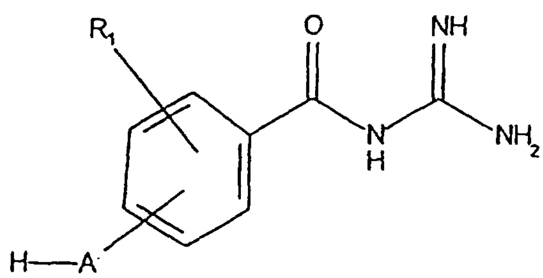

(XVIII)

wherein $R_1$ is as hereinbefore defined,
is reacted with a compound of formula

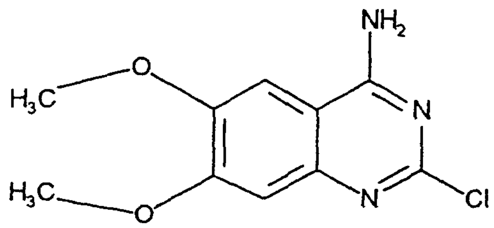

(XIX)

or

(c) a compound of formula

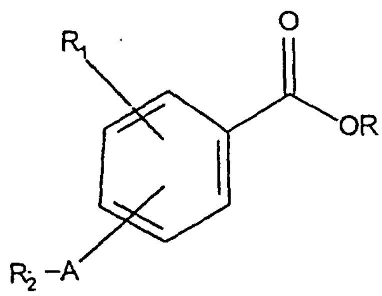

(X)

wherein R denotes a lower alkyl group, is reacted with guanidine,

and the products obtained are optionally separated into sterically different forms and/or any bases obtained are converted with acids into salts or any salts obtained are converted into free bases.

## Revendications

**1.** Composés de formule

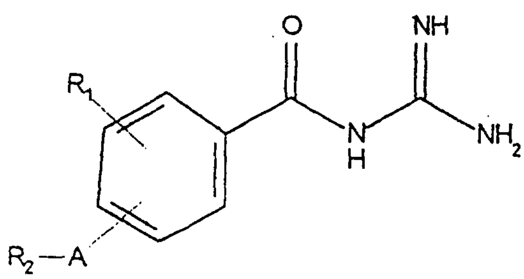

(I)

dans laquelle

A représente

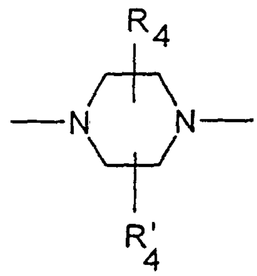

$R_1$ représente R'-$SO_2$- ou R'-NH-$SO_2$- (R' est alkyle en $C_1$-$C_5$, alkyle en $C_1$-$C_5$ substitué par halogène ou phényle, où le reste phényle peut comporter jusqu'à trois substituants du groupe d'halogène, alkyle en $C_1$-$C_4$ ou alcoxy en $C_1$-$C_4$), F, Cl ou $CF_3$ ;
$R_2$ représente un reste de formule

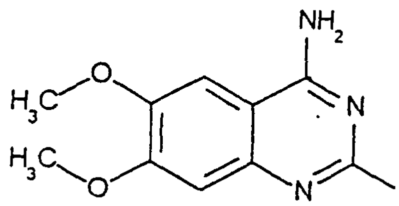

(II)

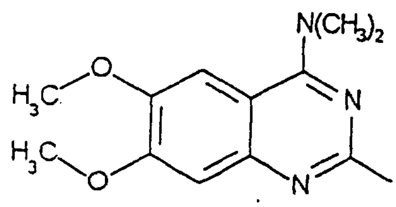

(III)

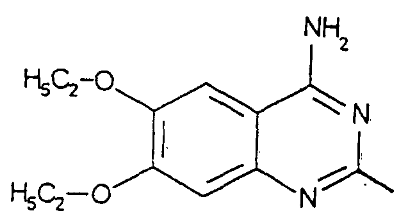

(IV)

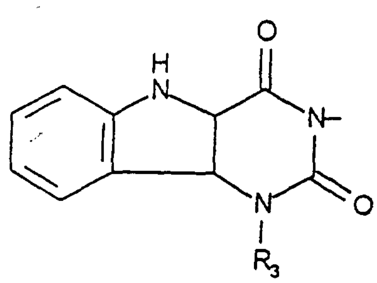

(V)

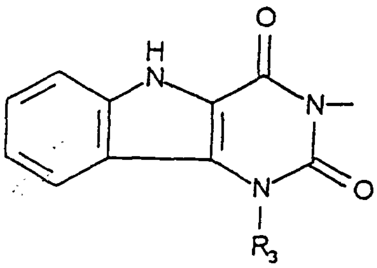

(VI)

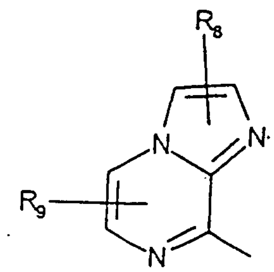

(VII)

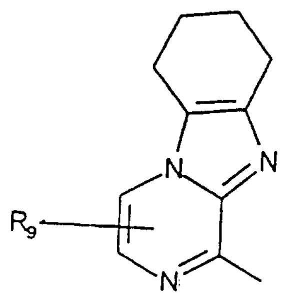

(VIII)

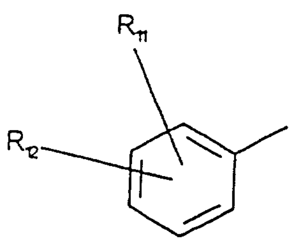

(IX)

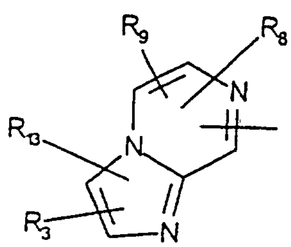

(X)

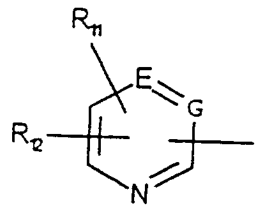

(XI)

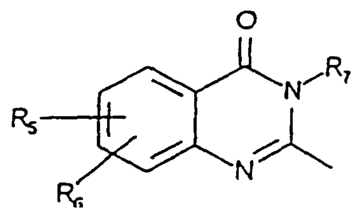

(XII)

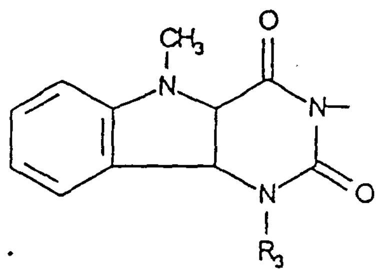

(XIII)

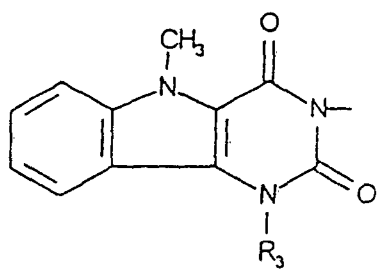

(XIV)

où

$R_3$, $R_4$ et $R_4$', qui peuvent être identiques ou différents, représentent l'hydrogène, alkyle en $C_1$-$C_4$, $R_4$ et $R_4$' peuvent aussi représenter phényle, benzyle et cycloalkyle en $C_3$-$C_7$,

$R_5$ et $R_6$, qui peuvent être identiques ou différents, représentent l'hydrogène, méthyle, méthoxy, hydroxyle ou halogène,

$R_7$ représente l'hydrogène, alkyle en $C_1$-$C_4$, benzyle ou benzyloxy,

$R_8$ et $R_9$, qui peuvent être identiques ou différents, représentent l'hydrogène, alkyle en $C_1$-$C_4$, phényle ou halogène,

$R_{10}$ représente l'hydrogène ou alkyle en $C_1$-$C_6$, qui peut aussi être substitué par phényle ou phényle substitué par méthyle, méthoxy ou halogène,

$R_{11}$ et $R_{12}$, qui peuvent être identiques ou différents, représentent l'hydrogène, méthyle, méthoxy, phényle, benzyle, nitro, cyano, halogène, trifluorométhyle, amino, $NR_{10}R_{13}$, 1-pyrrolidinyle, 1-pyrazolinyle, 1-imidazolidinyle, 1-pipéridinyle, 1-pipérazinyle ou carbamoyle, $R_{11}$ peut aussi représenter un cycle benzénique condensé qui peut comporter jusqu'à trois substituants du groupe de méthyle, méthoxy, halogène, $CF_3$ et CN,

$R_{13}$ représente l'hydrogène, alkyle en $C_1$-$C_4$, qui peut aussi être substitué par phényle, phénoxy, benzyloxy, ou halogène,

E et G, qui peuvent être identiques ou différents, représentent N ou CH,

où

les composés de formule I peuvent être sous forme de bases ou de sels et éventuellement sous forme des différents stéréoisomères ou isomères cis/trans ou des mélanges de tels isomères.

2. Composés de formule I selon la revendication 1 dans laquelle $R_2$ représente un reste des formules II à XV, $R_3$, $R_4$ et $R_4$', qui peuvent être identiques ou différents, représentent l'hydrogène ou un reste alkyle en $C_1$-$C_3$.

3. Composés de formule I selon la revendication 1 dans lesquels $R_2$ représente un reste de formule XI, E ou G représente un atome d'azote.

4. Composés de formule Ia

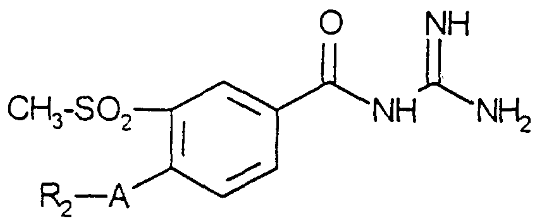

(I a)

selon l'une des revendications 1 à 3 dans laquelle A et $R_2$ ont la signification ci-dessus.

5. Médicament caractérisé par une teneur en un composé selon les revendications 1 à 4, outre des adjuvants et/ou supports courants.

6. Utilisation de composés selon les revendications 1 à 4 lors de la préparation de médicaments.

7. Composés selon les revendications 1 à 4 destinés à être utilisés dans le traitement de maladies qui sont en rapport avec des ischémies, dans le cas de l'infarctus cérébral, des troubles aigus et chroniques de l'irrigation du cerveau, pour la cardioprotection et pour éviter des lésions sur des greffes.

8. Procédé de préparation des composés selon les revendications 1 à 4 par des méthodes conventionnelles, caractérisé en ce que

(a) on fait réagir un composé de formule

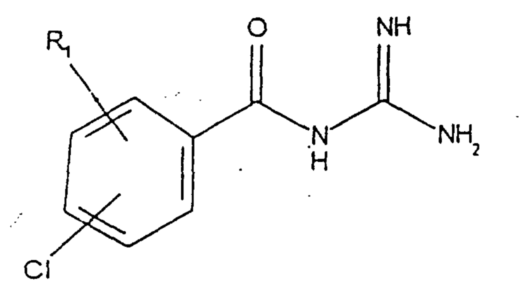

(XVI)

avec une amine de formule

$$R_2\text{-A-H} \qquad \text{(XVII)}$$

où $R_1$ et $R_2$ ont la signification ci-dessus,
ou en ce que

(b) on fait réagir un composé de formule

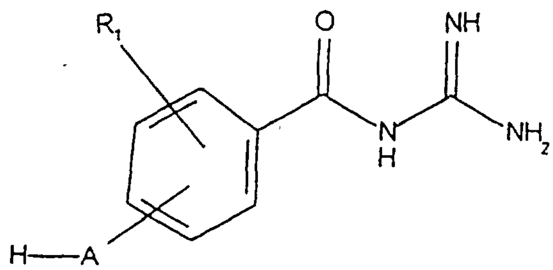

(XVIII)

où $R_1$ a la signification ci-dessus,
avec un composé de formule

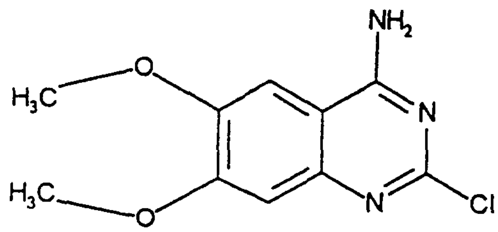

(XIX)

ou en ce que
(c) on fait réagir un composé de formule

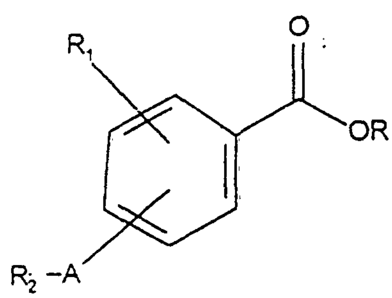

(X)

où R représente un reste alkyle inférieur
avec la guanidine

et on sépare les produits obtenus éventuellement en formes stériquement différentes et/ou, si on le souhaite, on convertit avec des acides les bases obtenues en sels ou les sels obtenus en bases libres.